# EUROPEAN PATENT APPLICATION

(11) **EP 0 834 303 A2**
(43) Date of publication of application: **08.04.1998**
(21) Application number: 97116359.7
(22) Date of filing: 19.09.1997
(51) Int. Cl.: A61K 7/13, A61K 7/06

(54) **Composition for coloring of human hair**

(30) Priority: 02.10.1996 DE 19640792; 02.10.1996 DE 19640831
(71) Applicant: Kao Corporation, Chuo-Ku Tokyo (JP)
(72) Inventor: Grit, Mustafa, Dr., 64665 Alsbach-Hähnlein (DE); Möhring, Hartmut, Dr., 64342 Seeheim-Jugenheim (DE); Schupp, Bettina, 64319 Pfungstadt (DE)
(74) Representative: Kindler, Matthias, Dr. Dipl.-Chem.

(57) **Abstract**

A composition for dyeing of human hair in an aqueous medium comprises
a) 0.0001% to 2.5% by wt. of at least one direct-acting, especially cationic hair dyestuff; and
b) 0.1% to 10% by wt. at least one cationic derived plant protein hydrolyzate and/or a compound of the general formula I 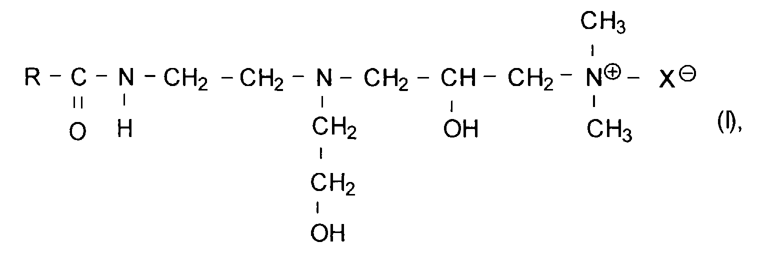 wherein R is a C₈-C₂₀-alk(en)yl group and X⁻ is an anion,
   calculated on the total composition.

These compositions not only provide improved and more intensive coloration, they also have an excellent conditioning effect.

## Description

This invention comprises a composition for the dyeing of human hair, providing superior coloring intensity while simultaneously conditioning the hair.

It is generally known that hair coloring compositions are classified into two categories, i.e., on the one hand permanent dyeing compositions comprising oxidizing dyestuff precursors, which together with oxidizing agents develop the hair coloration depending on the formula chosen; and, on the other hand, semi-permanent hair dyeing compositions comprising direct dyes which do not require any addition of an oxidizing agent to develop their dyeing effect. Accordingly, these dyeing results are less permanent than those obtained with permanent dyeing compositions.

These dyeing compositions on the basis of direct dyes are usually applied either as tinting shampoos, lotions or color setting lotions, optionally also as aerosol foam preparations.

The direct dyes used therein are normally of the cationic type; rinsing compositions also use cationic surfactants, particularly quaternary ammonium salts as essential ingredients.

The dyeing intensity and permanence achieved with these compositions is, however, not always satisfactory.

It has now been found that the dyeing intensity can be increased and an additional conditioning effect can be achieved when to such a composition, on an aqueous basis such as emulsion or dispersion, containing 0.0001 to 2.5 % by wt. of at least one direct dyestuff, preferably a cationic one, 0.1 to 10 % by wt., each calculated to the total composition, of at least one cationic plant protein hydrolyzate and/or a compound of the general formula I 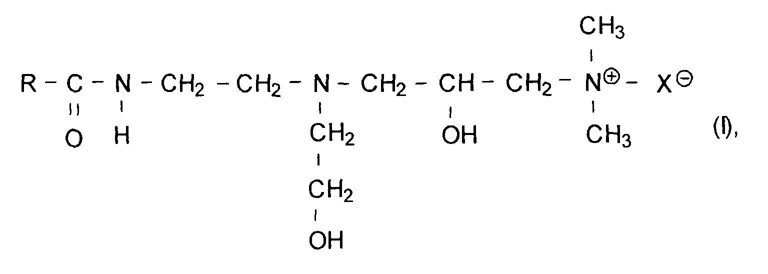 wherein R stands for a C₈ -C₂₀-alk(en)yl group and X⁻ is an anion, is added.

According to one of the preferred embodiments of the invention, the light fastness of the composition can be further improved by adding a preferably water-soluble UV-absorber containing anionic groups, whereby, surprisingly, a stabilization also takes place between the basically incompatible combination of cationic dyestuff/water-soluble UV-absorber with anionic groups.

Water-soluble UV-absorbers with anionic groups are already known per se. Due to their substantivity, they are basically suitable for improving the light fastness of colorations achieved by the use of hair dyes containing direct-acting dyestuffs.

However, it was not possible to incorporate these water-soluble, UV-absorbing substances containing anionic groups into customary cationic direct-acting hair dyestuff compositions, as obviously an interaction took place between the two substances, leading to an instable composition.

It was then also found, and this is also subject of a preferred embodiment of this invention, that stable hair dyeing compositions on an aqueous basis containing direct-acting cationic hair dyestuffs can be obtained, which produce on the hair lustrous, durable, expressive and light-fast colorations, by the addition of a combination of 0.1% to 5% by wt. of at least one water-soluble, an anionic group, particularly a sulfate, carboxyl, or phosphate group bearing UV-absorbing agent, and 0.1% to 10% by wt. of at least one cationic derived plant protein hydrolyzate, in particular on the basis of a wheat protein, and/or a quaternary ammonium compound of formula I.

Cationic derived plant protein hydrolyzates suitable within the frame of the invention are in particular those from wheat protein, almond protein, soya protein, pea protein, corn protein, oat protein, peanut protein, rice protein, bean protein, etc..

The protein hydrolyzates, preferably produced through enzymatic hydrolysis of the respective plant proteins, are cationized in the basically known manner by conversion with a long chain quaternary ammonium salt, as described for example in DE 95 05 005 C1.

The cationized plant protein hydrolyzates described therein are suitable for application in the semi-permanent and permanent hair dyeing compositions according to the invention just as the cationic plant protein hydrolyzates known under the trade names "Gluadin™ 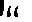 , i.e. "Gluadin™ WQ 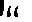 , "Quat-Soy™" and "Quat-Wheat ™" and the CTFA names Soyadimonium Hydroxypropyl Hydrolyzed Wheat Protein, Cocodimonium Hydrorypropyl Hydrolyzed Wheat Protein, and Hydroxypropyl Trimonium Hydrolyzed Wheat Protein.

The compounds in formula I and their production are known from the EP-A 472 107.

As has already been stated, the proportion of the named quaternary compounds amounts to 0.1% up to 10% by wt., preferably 0.25% to 7.5% by wt., in particular 0.5% to 5%, especially 0.75% to 2.5% by weight, calculated to the total composition.

As already explained, in order to further improve the light-fastness of the achieved hair coloration, the compositions according to the invention may contain a water-soluble UV-absorber added preferably in a quantity of 0.1% to 5%, in particular 0.25% to 2.5% by weight, calculated to the total composition.

Specially suitable water-soluble UV-absorbers with anionic groups are, for example, 5-benzoyl-4-hydroxy-2-methoxybenzene sulfonic acid (Benzophenone-4), its sodium salt (Benzophenone-5) and 2.2'-dihydroxy-4.4'-dimethoxy-3.3'-disulfobenzophenone or the disodium salt thereof (Benzophenone-9) as well as phenyl benzimidazole sulfonic acid (Eusolex ™ 232); however, it is also possible to incorporate other UV-absorbers.

The preferred weight ratio of cationic plant protein derivative or compound of formula I to water-soluble anionic UV-absorber is to be found in the range of approximately 1 : 1 to approximately 3 : 1

The proportion of the direct-acting dyestuffs in the compositions according to the invention is variable and lies between approximately 0.0001% to approximately 2.5%, preferably 0.001 to 1, in particular 0.01 to 0.5% by weight of the total composition.

In principle all cationic dyestuffs proposed for this purpose are preferred as directacting hair dyestuffs.

Particularly preferred are the so-called 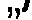 Arianor 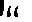 dyestuffs; see K. Schrader, 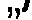 Grundlagen und Rezepturen der Kosmetika 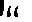 , 2nd Ed. (1989). p. 811.

Especially suitable basic (cationic) dyestuffs are:
- Basic Blue 6,: C.I.-No. 51,175;
- Basic Blue 7,: C.I.-No. 42,595;
- Basic Blue 9,: C.I.-No. 52,015;
- Basic Blue 26,: C.I.-No. 44,045;
- Basic Blue 41,: C.I.-No. 11,154;
- Basic Blue 99,: C.I.-No. 56,059;
- Basic Brown 4,: C.I.-No. 21,010;
- Basic Brown 16,: C.I.-No. 12,250;
- Basic Brown 17,: C.I.-No. 12,251;
- Natural Brown 7,: C.I.-No. 75,500;
- Basic Green 1,: C.I.-No. 42,040;
- Basic Green 4,: C.I.-No. 42,000;
- Basic Red 1,: C.I.-No. 45,160;
- Basic Red 2,: C.I.-No. 50,240;
- Basic Red 22,: C.I.-No. 11,055;
- Basic Red 76,: C.I.-No. 12,245;
- Basic Violet 1,: C.I.-No. 42,535;
- Basic Violet 3,: C.I.-No. 42,555;
- Basic Violet 10,: C.I.-No. 45,170;
- Basic Violet 14,: C.I.-No. 42,510;
- Basic Yellow 2,: C.I.-No. 41,000;
- Basic Yellow 57,: C.I.-No. 12,719.

Of course, the (additional) use of appropriate direct plant dyestuffs is also possible.

The compositions according to the invention may also preferably contain at least one cationic surfactant in addition to the cationic plant protein hydrolyzates or compounds of formula I, in particular in a quantity of 0.1% to 7.5%, preferably 0.25% to 5%, most preferably 0.5% to 2.5% by wt. of the total composition.

Suitable long-chain quaternary ammonium compounds, which may be used as cationic surfactants either alone or in admixture, are in particular cetyl trimethyl ammonium chloride, dimethyl dicetyl ammonium chloride, trimethyl cetyl ammonium bromide, stearyl trimethyl ammonium chloride, dimethyl stearyl benzyl ammonium chloride, benzyl tetradecyl dimethyl ammonium chloride, dimethyl dihydrogenated stearic ammonium chloride, lauryl pyridinium chloride, lauryl dimethyl benzyl ammonium chloride, lauryl trimethyl ammonium chloride, tris(oligo oxyethyl) alkyl ammonium phosphate, cetyl pyridinium chloride, etc..

In principle, all quaternary ammonium compounds listed in the 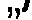 CTFA International Cosmetic Ingredient Dictionary 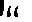 , Fourth Ed. (1991) under the common name 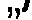 Quaternium 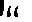 are suitable.

It is also possible to incorporate amphoteric surfactants in subordinate amounts, in particular those belonging to the betaine type.

Nonionic surfactants, in particular admixed with cationic surfactants, may also be used, such as amine oxides in a proportion of approximately 0.25% to approximately 5%, preferably approximately 0.5% to approximately 3.5% by weight, calculated to the total composition.

For a substantial period of time such amine oxides have been state of the art, e.g. C₁₂-C₁₈-alkyl dimethyl amineoxides such as lauryl dimethyl amineoxide, C₁₂-C₁₈ -alkyl amidopropyl- or ethyl amineoxides, C₁₂-C₁₈ -alkyl di(hydroxyethyl)-or (hydroxypropyl) amineoxides, or also amineoxides with ethylene oxide and/or propylene oxide groups in the alkyl chain.

Suitable surfactants are further the known C₈C₁₈-alkyl polyglucosides, in particular with a polycondensation degree of 1.2 to 3.

On the other hand, the presence of a quantifiable proportion of anionic surfactants is unsuitable and undesirable.

The composition may naturally also contain the components customarily found in such conditioning compositions; to avoid repetition, reference is again made to K. Schrader, 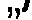 Grundlagen und Rezepturen der Kosmetika 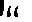 , 2nd Ed. (1989), pp. 722 - 771.

These are, for example, synthetic or natural hair conditioning polymers, preferably in a proportion of 0.1% to 2.5%, in particular 0.25% to 1.5% by wt., calculated to the total composition.

Suitable cationic polymers are known. In addition to the well established quaternary cellulose derivatives of the type 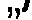 Polymer JR 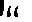 , in particular quaternized homo- and copolymers of dimethyl diallyl ammonium chloride, as they are on the market under the trade name 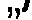 Merquat^{R} 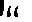 , quaternary vinyl pyrrolidone copolymers, in particular with dialkyl aminoalkyl (meth)acrylates known under the trade name 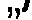 Gafquat^{R} 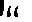 , copolymers of vinyl pyrrolidone and vinyl imidasolinium methochloride offered on the market under the trade name 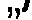 Luviquat^{R} 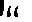 , polyamino-polyamide derivatives, e.g. copolymers of adipic acid dimethyl amino hydroxypropyl diethylene triamine sold under the name 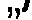 Cartaretine^{R} F 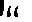 , as well as bisquaternary long-chain ammonium compounds of the urea structure described in the US patent 4 157 388, sold on the market under the trade name 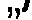 Mirapol^{R} A 15 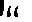 .

In this context reference is also made to the cationic polymers described in German Patent Application Nos. 25 21 960, 28 11 010, 30 44 738 and 32 17 059 and the products disclosed on pages 3 to 7 of EP-A 337 354. Mixtures of different cationic polymers may also be used.

Nonionic polymers may also be used in place of cationic polymers or in combination with these. Suitable nonionic polymers, above all, are vinyl pyrrolidone homo- and copolymers, in particular polyvinyl pyrrolidone itself, copolymers of vinyl pyrrolidone and vinyl acetate or terpolymers of vinyl pyrrolidone, vinyl acetate and vinyl propionate, e.g. sold by BASF under the trade name 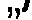 Luviskol^{R} 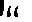 .

However, (co-)polymers of the various acrylic and methacrylic esters, acrylamide and methacrylamide, e.g. polyacrylamide having a molecular weight of more than 100,000, dimethyl hydantoin formaldehyde resins, etc., may also be used. Mixtures of different nonionic polymers are, of course, also possible.

Finally, amphoteric polymers, e.g. the copolymers of N-octyl acrylamide, N-butyl aminoethyl methacrylate and acrylic acid sold under the trade name 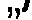 Amphomer^{R} 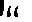 , are also suitable.

The hair dying compositions according to the invention may comprise the additives usually found in such compositions, whose type and character depend on the form of application. These are fats, fatty alcohols, emulsifiers, pH-regulants, solvents and compounding agents, solubilizers, preservatives, perfumes, etc..

Suitable fats and oils, which also include waxes, are in particular natural oils such as avocado oil, coconut oil, palm oil, sesame oil, peanut oil, whale oil, sunflower seed oil, almond oil, peach kernel oil, wheat germ oil, macadamia nut oil, night primrose oil, jojoba oil, castor oil, or also olive or soybean oil, lanolin, and its derivatives, also mineral oils such as paraffin oil and vaseline.

Synthetic oils and waxes are e.g. silicone oils, polyethylene glycols, etc..

Other suitable hydrophobic components are in particular fatty alcohols, preferably those with about 8 to 22 carbon atoms in the molecule such as myristyl, cetyl, stearyl alcohol, wax alcohols and fatty acid esters such as isopropyl myristate, palmitate, stearate and isostearate, oleyl oleate, isocetyl stearate, hexyl laurate, dibutyl adipate, dioctyl adipate, myristyl myristate, oleyl erucate, polyethyleneglycol and polyglyceryl fatty acid esters such as PEG-7-glyceryl cocoate, cetyl palmitate, etc..

These hydrophobic components are incorporated into the compositions according to the invention in a proportion of preferably from about 0.5% to about 10%, particularly from about 1% to 7.5%, optimally from about 1.5% to 5% by wt., calculated to the total composition.

A summary of the production of such compositions can be found in K. Schrader's monography to which reference has already been made, pp. 798 to 815, in particular pp. 804 ff.

The dyeing compositions according to the invention are formulated as emulsions, dispersions or optionally thickened, e.g. as gels, and may also be packed as aerosol foams. These preparations and their manufacture are generally known to the expert and therefore require no further explanation.

The pH-value of the hair dyeing compositions according to the invention is preferably from 3 to 7, in particular between 4 and 6.

The following Examples illustrate the composition of preparations according to the invention:

Color gloss rinses of the following compositions were prepared by simple admixture of the ingredients.

| | **Example Nos.** | | | | |
|---|---|---|---|---|---|
| **Ingredients:** | **1** | **2** | **3** | **4** | |
| 1,2-Propanediol | 3.00 | 3.00 | 3.00 | 3.00 | |
| Cetyl stearyl alkohol | 1.25 | 1.25 | 1.25 | 1.25 | |
| Hydroxyethyl cellulose | 1.00 | 1.00 | 1.00 | 1.00 | |
| Silicone oil | 0.20 | 0.20 | 0.20 | 0.20 | |
| Methyl parabene | 0.20 | 0.20 | 0.20 | 0.20 | |
| Cetyl trimethyl ammonium chloride | 0.20 | 0.20 | 0.40 | 0.20 | |
| Dicetyl dimethyl ammonium chloride | 0.50 | 0.60 | 0.25 | 0.50 | |
| Henna extract | 1.00 | - | 1.00 | - | |
| Camomile extract | 1.00 | 1.00 | 1.00 | 1.00 | |
| Nut extract | - | 1.00 | - | 1.00 | |
| Perfume | 0.50 | 0.50 | 0.50 | 0.50 | |
| Benzophenone-4 | 0.60 | 0.60 | - | - | |
| Cationic plant hydrolyzate (Gluadin™ WQ) | - | 1.00 | - | 1.00 | |
| Compound of formula I (R=C₁₃H_{27;} X=Cl⁻) | 1.00 | - | 1.00 | - | |
| Basic Red 2 | 0.50 | - | 0.50 | - | |
| Basic Brown 16 | - | 0.20 | - | 0.50 | |
| Citric acid | q.s. | q.s. | q.s. | q.s. | |
| Water | @ 100,00 | @ 100,00 | @ 100,00 | @ 100,00 | % by wt. |
| **Hair coloring achieved:** | **Dark red** | **Dark brown** | **Dark red** | **Dark brown** | |

Omitting the quaternary ammonium compounds according to formula I or the cationic plant protein hydrolyzate in the preparation of Examples 1 and 2 resulted in instable aqueous compositions, i.e. the formation of sediments after about 24 hours storage.

Omitting the quaternary ammonium compound according to formula I or the cationic plant protein hydrolyzate in the compositions according to Examples 3 and 4 resulted in colorations which were substantially reduced in color brilliance and permanence compared to the colorations achieved with the compositions according to the invention.

A particular useful embodiment of the invention are tinting shampoos on an aqueous basis comprising at least one anionic, nonionic and /or amphoteric (zwitterionic) surfactant, with good conditioning and coloration properties, comprising a combination of
a) 0.001% to 2.5% by wt. of at least one direct-acting hair dyestuff; and
b) 0.1% to 10% by wt. of at least one cationic protein hydrolyzate, each calculated to the total composition.

Hair treated with such a shampoo not only has an intensive, long-lasting, stable coloration, but is also provided with volume, body, improved gloss and easy combability in comparison to conventional shampoos comprising no cationic plant protein hydrolyzates.

The tinting shampoos comprise anionic, nonionic and/or amphoteric (zwitterionic) surfactants, preferably in an amount from 2.5% to 30% by wt., calculated to the total composition

Suitable anionic surfactants are in particular those of the sulfate, sulfonate, carboxylate or alkyl phosphate type generally used in these products, e.g. the known C₁₀-C₁₈-alkyl sulfates, and in particular the respective ether sulfates, e.g. C₁₂-C₁₄-alkyl ether sulfate, lauryl ether sulfate, especially with 1 to 4 ethylene oxide groups in the molecule, furthermore monoglyceride sulfates, fatty acid amide sulfates, which are obtained by ethoxylation and subsequent sulfation of fatty acid alkanolamides, and their alkali salts as well as the salts of long-chain mono- and dialkyl phosphates represent the mild, skin-compatible detergents.

Further anionic surfactants also suitable within the scope of the invention are α-olefin sulfonates or the salts thereof, in particular also alkali salts of sulfosuccinic acid semi-esters, for example the disodium salt of the mono-octyl sulfosuccinate and alkali salts of long-chain monoalkyl ethoxy sulfosuccinates, e.g. disodium lauryl ether sulfosuccinate, which are especially preferred within the scope of the invention.

It is also preferable to use admixtures of several anionic surfactants, for example a mixture of an α-olefin sulfonate and a sulfosuccinate, preferably in a proportion of 1 : 3 to 3 : 1.

In admixture with anionic surfactants also protein fatty acid condensation products known per se can be used, in particular in quantities from about 0.5% to 5%, preferably 1% to 5% by wt., calculated to the total composition.

Suitable carboxylates are in particular polyalkyl ether carboxylic acids and the salts thereof of the formula R-(C₂H₄O)ₙ-O-CH₂-COOX, and alkyl am do ether carboxylic acids of the formula 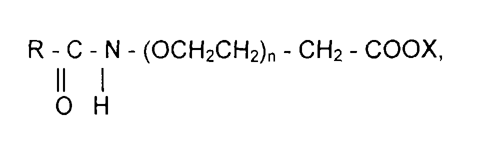 wherein R is a C₈-C₂₀-alkyl group, preferably a C₁₂-C₁₄-alkyl group, n is a number from 1 to 20, preferably 2 to 15, in particular 2.5 to 12, and X is H or preferably a cation of the sodium, potassium, magnesium and ammonium group, which can be substituted by an alkyl or hydroxyalkyl group.

Such products have been known and on the market for some time, for example under the trade name "AKYPO" and "AKYPO-SOFT™".

Furthermore, an overview of the anionic surfactants used in liquid shampoos can be found in K. Schrader's monography, "Grundlagen und Rezepturen der Kosmetika", 2nd Ed. (1989, Hüthig Buchverlag), pp. 683 to 691.

The total quantity of surfactants used in the liquid tinting shampoos according to the invention is preferably between 2.5% and 30% by wt., in particular between 5% to 20% by wt., especially ranging from about 5% to about 15% by wt., calculated to the total composition.

According to a further preferred embodiment of the invention, the liquid tinting shampoo comprises nonionic surfactants, preferably in admixture with anionic surfactants, in a quantity of about 1% to about 15% by wt., preferably at least one nonionic surfactant in about 2.5% to about 10% by wt., calculated to the total composition.

One preferred nonionic surfactant therein refers to the type of alkyl polyglucosides of the general formula

R - O - (CH₂CH₂O)ₙ-Gₓ ,

wherein R is an alkyl group with 8 to 18 carbon atoms, G is a sugar residue with 5 to 6 carbon atoms, n is a number from 0 to 10, and x is number between 1.2 and 2.5.

These alkyl polyglucosides have recently become known especially as excellent, skin compatible, foam improving agents in liquid laundry and body cleaning agents.

Further components of nonionic surfactants are long-chain fatty acid mono- and dialkanolamides, for example coconut fatty acid monoethanolamides and myristine fatty acid monoethanolamides, which can also be used as foam boosters, as well as the various sorbitan esters, such as polyethylene glycol sorbitan stearic acid ester, fatty acid polyglycol ester or also mixed condensates of ethylene oxide and propylene oxide, as they are on the market for example under the trade name "Pluronics".

C₈-C₁₈- fatty alcohol ethoxylates are also suitable, for example those comprising 10 to 20 ethylene oxide groups per molecule, and ethoxylated C₈-C₁₈- fatty acid monoalkanol- amides with 1 to 20 ethylene oxide groups per molecule.

Admixtures of anionic surfactants and alkyl polyglucosides, the preferred nonionic surfactants within the scope of the invention, as well as their use in liquid tinting shampoos, are known per se, for example from EP-A 70 074.

The alkyl polyglucosides described therein are basically also suitable within the scope of the present invention; as are the admixtures of sulfosuccinates and alkyl polyglucosides known from EP-A 358 216.

It is also possible to integrate surface-active amineoxides, for example in an amount ranging from about 0.25% to about 5%, preferably about 0.5% to about 3.5% by wt., calculated to the total composition.

Such amine oxides have been state of the art for a substantial period of time, for example C₁₂-C₁₈-alkyl dimethyl amineoxides such as lauryl dimethyl amineoxide, C₁₂-C₁₈-alkyl amidopropyl or -ethyl amineoxides, C₁₂-C₁₈-alkyl di(hydroxyethyl) or (hydroxypropyl)amineoxides, or also amineoxides with ethyleneoxide- and/or pro-pyleneoxide groups in the alkyl chain.

The compositions according to the invention preferably also comprise amphoteric (zwitterionic) surfactants in an amount of about 0.1% to about 15%, preferably about 0.5% to about 10% by wt., in particular up to 7.5% by wt., calculated to the total composition, preferably as main component, for example in admixture with anionic and/or nonionic surfactants. As amphoteric surfactants the various known betaines such as fatty acid amidoalkyl betaines and sulfobetaines, for example lauryl hydroxy sulfobetaine and long-chain alkylamino acids have proved as equally suitable.
In detail, it is possible to use betaines of the structure 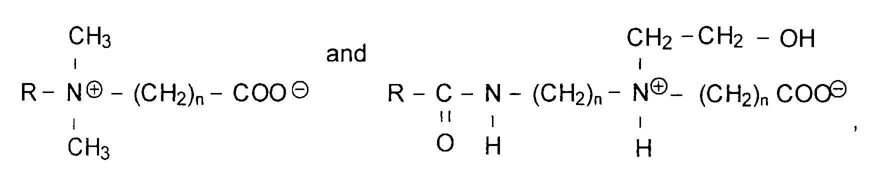 wherein R is a C₈-C₁₈-alkyl group and n is 1 to 3,
sulfobetaines of the structure 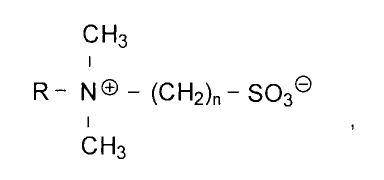 wherein R is a C₈-C₁₈-alkyl group and n is 1 to 3,
amide alkyl betaines or sulfobetaines of the structure 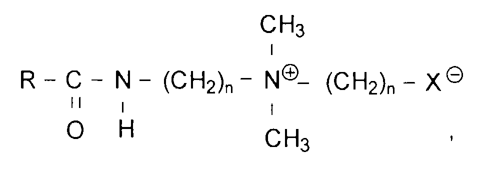 wherein R is a C₈-C₁₈-alkyl group, X is a carboxyl- or sulfogroup, and n is 1 to 3, and long-chain alkyl amine carboxylic acids are used.

The cationic wheat protein hydrolyzates used in the invention are those described hereinbefore.

The proportion thereof in the shampoos is from 0.1% to 10%, preferably 0.5% to 7.5%, in particular 1% to 5% by wt., calculated to the total composition.

When an anionic surfactant is comprised in the tinting shampoo according to a preferred embodiment of the invention, the weight proportion of anionic surfactant to cationic plant protein hydrolyzate ranges between 1 : 3 and 15 : 1, in particular between 1 : 2 and 10 : 1.

It is self-understood that the liquid tinting shampoos may also comprise all the substances customarily integrated into such products.

As examples for such substances we would name complex formers, dyestuffs, preservatives, pH-regulants, viscosity modifiers such as inorganic salts as far as these are not already present in the original surfactant mixtures, fragrances, pearl gloss agents, thickening agents, moisture retainers, plant and animal oils such as jojoba oil, etc..

A list of such additives can also be found in Schrader, I.c., on pp. 695 to 722.

Additives especially suited for shampoos are hair conditioning agents. If compatible with the other ingredients, such additives are anionic, cationic, amphoteric and/or nonionic polymers, preferably in an amount between 0.1% to 2%, in particular 0.25% to 1.25% by wt., calculated to the total composition.

Such polymers are also listed hereinbefore.

The use of cationic polymers together with alkyl polyglucoside surfactants is already known from EP-A 337 354; the cationic polymers listed therein on pp. 3 to 7 are also suitable as conditioning additives in the compositions according to the invention.

Further conditioning additives are the known non-cationic protein hydrolyzates, e.g. in an amount from 0.25% to 5% by wt., preferably 0.5% to 2.5% by wt., calculated to the total composition.

Also suited is water-soluble collagen or water-soluble collagen derivatives.

The direct-acting hair dyestuffs are known per se. Their type and quantity depends on the desired coloration; the quantities generally used range from 0.001% and 2.5%, in particular from about 0.05% to 1% by wt., whereby cationic dyestuffs are preferred.

Suitable cationic dyestuffs are already listed hereinbefore.

Although less preferred, suitable anionic dyestuffs may also be used where appropriate. Examples are:
- Acid Black 1,: C.I.-No. 20,470;
- Acid Blue 9,: C.I.-No. 42,090;
- Acid Blue 74,: C.I.-No. 73,015;
- Acid Red 18,: C.I.-No. 16,255;
- Acid Red 27,: C.I.-No. 16,185;
- Acid Red 87,: C.I.-No. 45,380;
- Acid Red 92,: C.I.-No. 45,410;
- Acid Violet 43,: C.I.-No. 60,730;
- Acid Yellow 1,: C.I.-No. 10,316;
- Acid Yellow 23,: C.I.-No. 19,140;
- Acid Yellow 3,: C.I.-No. 47,005;
- D&C Brown No.1,: C.I.-No. 20,170;
- D&C Green No.5,: C.I.-No. 61,570;
- D&C Orange No.4,: C.I.-No. 15,510;
- D&C Orange No.10,: C.I.-No. 45,425:1;
- D&C Orange No.11,: C.I.-No. 45,425;
- D&C Red No.21,: C.I.-No. 45,380:2;
- D&C Red No.27,: C.I.-No. 45,410:1;
- D&C Red No.33,: C.I.-No. 17,200;
- D&C Yellow No.7,: C.I.-No. 45,350:1;
- D&C Yellow No.8,: C.I.-No. 45,350;
- FD&C Red No.4,: C.I.-No. 14,700;
- FD&C Yellow No.6,: C.I.-No. 15,985.

A list of direct-acting dyestuffs can also be found in Schrader, I.c., pp. 800 - 805. Naturally, it is also possible to integrate direct-acting natural dyestuffs, such as e.g. henna, chamomile, madder root, sandalwood or walnut. In addition, optical brighteners, for example Fluorescent Brightener 140, can also be integrated to achieve lighter shades.

The following Examples serve to illustrate this preferred embodiment of the invention.

The products according these Examples are manufactured by admixing the individual components in water, whereby it is also possible to use pre-mixtures of the various ingredients.

| | **Example-No.** | | | |
|---|---|---|---|---|
| **Ingredients** | **5** | **6** | **7** | **8** |
| Cocoamido polyether carboxylic acid ( 4 EO-units), sodium salt | 1.00 | 1.00 | 1.00 | 1.00 |
| Decyl polyglucoside(P.D.: ≈ 1,5) | 3.00 | 3.00 | 3.00 | 3.00 |
| Cocoamidopropyl betaine | 5.00 | 5.00 | 5.00 | 5.00 |
| Lauryl hydroxy sultaine | 0.50 | 0.50 | 0.50 | 0.50 |
| Polyacrylic acid salt (Carbopol™ 2020) | 0.30 | 0.30 | 0.30 | 0.30 |
| Polyquaternium-6 | 0.50 | 0.50 | 0.50 | 0.50 |
| HC Red 3 | 0.10 | 0.05 | 0.02 | 0.10 |
| Cationic wheat protein hydrolyzate (Gluadin™ WQ) | 0.50 | 0.50 | 0.50 | - |
| Perfume, preservative | q.s. | q.s. | q.s. | q.s. |
| Water | @ 100.00 %by wt. | @ 100.00 %by wt. | @ 100,00 %by wt. | @ 100,00 %by wt. |

5 strains each of bleached buffalo hair were treated with these shampoo compositions. After 20 minutes processing at 40 °C, with subsequent rinsing, the Minolta CR 200 was used to measure the respective ΔE-values for the color brilliance achieved with each red coloration. The results clearly show the superiority of the cationic wheat protein hydrolyzates comprised in the composition according to the invention.

| **ΔE-Values** | |
|---|---|
| **Composition No.** | **ΔE** |
| 1 | 38.0 |
| 2 | 27.1 |
| 3 | 21.8 |
| 4 | 7.5 |
| (The higher ΔE-value, the better the achieved color brilliance). | |

### Example 9

| | | |
|---|---|---|
| Cocoamido polyether carboxylic acid (3-4 EO-units), sodium salt | 1.00 | (% by wt.) |
| Sodium lauryl ether sulfate | 7.00 | |
| Polysorbate 20 | 1,00 | |
| Dimethyl lauryl amine oxide | 2.00 | |
| Lauryl amidopropylbetaine | 3.00 | |
| Pearl gloss agent (Euperlan® PK 900) | 2.00 | |
| PEG-4-Rape seed oil monoethanolamide | 2.00 | |
| Dimethicone copolyol | 1.00 | |
| Lauryl dimonium hydroxypropyl wheat protein hydrolyzate | 1.00 | |
| Cationic cellulose derivate(Polymer® JR 400) | 0.50 | |
| Basic Brown 17 | 0.001 | |
| Basic Yellow 57 | 0.01 | |
| C.I. Fluorescent Brightener 140 | 0.08 | |
| Perfume, preservative | q.s. | |
| Water | @ 100.00 | |

Application of this shampoo with its good lather qualities resulted in a light-blond, glossy hair shade.

| | **Examples No.** | | | | | | |
|---|---|---|---|---|---|---|---|
| **Ingredients** | 10 | 11 | 12 | 13 | 14 | 15 | 16 |
| Cocoamidopropyl betaine | 10.00 | 7.00 | 6.00 | 7.50 | - | 10.00 | 8.50 |
| C₁₂-C₁₄-alkyl polyglucoside (P.D- ^{∼} 1.5) | 3.00 | 6.00 | 5.00 | 5050 | 8-00 | 6.00 | 4.00 |
| Lauryl ether (-2.5-)sulfate, sodium salt | - | 1.50 | - | 1.10 | - | - | - |
| Lauryl hydroxy sultaine | - | 0.60 | 0.60 | 0.60 | - | 1.00 | 2.00 |
| Laureth -16 | ----- | | -- | - | -3.50 | - | - |
| Laureth-4.5-carboxylate, sodium salt | - | - | - | 1.10 | - | - | - |
| Lauryl alcohol polyglycol ether sulfosuccinate | - | -1.00 | - | - | - | - | - |
| Dimethyl lauryl amine oxide 1.50 | 2.00 | 1.50 | 1.50 | 1.50 | - | 2.00 | 3.00 |
| Lauryl amido-PEG-4-carboxylic acid, sodium salt | 0.50 | - | - | - | 3.00 | - | - |
| Cationic wheat protein hydrolyzate (Gluadin™ WQ) | 0.50 | 1.00 | 1.00 | 1.00 | 3.20 | 0.60 | 1.00 |
| Sorbitan-400-trioleate | 1.00 | 1.00 | 1.00 | 1.00- | 1.00 | 1.00 | 1.00 |
| PEG-120-methyl glucose dioleate | - | - | - | - | 1.00 | - | - |
| Polyquaternium-6 | 0.50 | - | 0.50 | 0.50 | 1.00 | - | - |
| Polyquaternium-7- | - | 0.50 | - | - | - | - | - |
| Polyquaternium-24 | - | - | - | - | 0.60 | - | - |
| Walnut oil | - | - | - | - | - | 0.50 | - |
| Solubilizer (Cremophor™ RH60) | 0.50 | 0.50 | 0.50 | 0.50 | 0.50 | 0.50 | 0.50 |
| Perfume oil | 0.50 | 0.50 | 0.50 | 0.50 | 0.50 | 0.50 | 0.50 |
| Henna extract | - | - | - | - | 3.00 | - | - |
| Preservative | 0.10 | 0.10 | 0.10 | 0.10 | 0.50 | 0.10 | 0.10 |
| Optical brightener (Tinopal™ SWN) | - | - | - | 0.10 | - | - | - |
| HC-Red 3 | 0.02 | - | 0.02 | - | - | 0.02 | 0.01 |
| Basic Red 76 | 0.04 | - | - | - | - | - | - |
| Basic Yellow 57 | - | - | 0.05 | - | - | - | - |
| Basic Blue 99 | - | - | - | - | - | - | 0.01 |
| Disperse Black 9 | - | 0.10 | - | - | - | 0.10 | - |
| Basic Brown 16 | - | - | - | - | - | 0.03 | - |
| Water | @ 100% by wt. | @ 100% by wt. | @ 100% by wt. | @ 100% by wt. | @ 100% by wt. | @ 100% by wt. | @ 100% by wt. |
| **Hair coloring achieved** | red shade for brown hair | yellow shade for blond hair | red-gold shade for blond hair | bright ener for blond hair | reddish shade for blond hair | red-brown shade for dark-blond hair | anti-yellow effect for gray hair |

## Claims

1. Composition for dyeing of human hair in an aqueous medium, comprising
a) 0.0001% % to 2.5% by wt. of at least one direct-acting hair dyestuff; and
b) 0.1 to 10% by wt. of at least one cationic derived plant protein hydrolyzate and/or a
compound of the general formula I 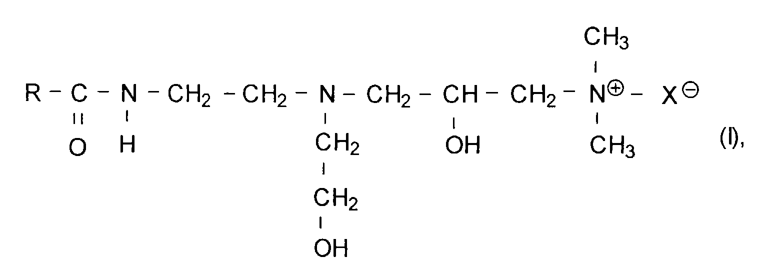 wherein R is a C₈-C₂₀-alk(en)yl group and X⁻ is an anion,
all percentages calculated to the total composition.

2. Composition according to claim 1, characterized in that the cationic derived plant protein hydrolyzate is a wheat protein hydrolyzate.

3. Composition according to claims 1 or 2, comprising at least 0.1% to 15% by wt., calculated to the total composition, of a cationic, amphoteric (zwitterionic) and/or nonionic surfactant.

4. Composition according to claim 3, comprising at least 0.1% to 7.5% by wt, calculated to the total composition, of at least one surface-active, long-chain quaternary ammonium compound.

5. Composition according to claim 3, comprising at least 0.1% to 10% by wt., calculated to the total composition, of at least one amphoteric (zwitterionic) surfactant.

6. Composition according to claim 5, comprising a long-chain betaine, sulfobetaine, and/or long-chain alkyl amino carboxylic acids.

7. Composition according to claim 3, comprising C₈-C₂₀-alkyl polyglucosides with a condensation degree of 1.25 to 2.5, surface-active alkyl amine oxides, and/or C₈-C₂₀ -fatty alcohol ethoxylates.

8. Composition according to one or more of claims 1 to 7, comprising 0.1% to 5% by wt. of at least one water-soluble UV-absorbing substance containing an anionic group.

9. Composition according to one or more of claims 1 to 8, having a pH-value of 3.5 to 7.

10. Composition according to claim 1, being a tinting shampoo on an aqueous basis, comprising at least one anionic, nonionic, and/or amphoteric surfactant and a combination of
a) 0.001% to 2.5% by weight of at least one direct-acting hair dyestuff, and
b) 0.1% to 10% by weight of at least one cationic plant protein hydrolyzate, each calculated to the total composition.

11. Tinting shampoo according to claim 10, comprising a mixture of at least one amphoteric surfactant and at least one nonionic surfactant.

12. Tinting shampoo according to claims 10 or 11, comprising at least one anionic surfactant and at least one cationic plant protein hydrolyzate in a weight proportion of 1 : 3 to 15 : 1.

13. Tinting shampoo according to claim 12, characterized by a weight proportion between anionic surfactant to cationic plant protein derivative of 1 : 2 to 10 : 1.

14. Tinting shampoo according to one or more of claims 10 to 13, comprising 1% to 15% by weight of at least one amphoteric surfactant, calculated to the total composition.

15. Tinting shampoo according to claim 14, comprising as amphoteric surfactant a C₈-C₁₈-alkyl amidopropyl betaine.

16. Tinting shampoo according to one or more of claims 10 to 15, comprising as cationic protein hydrolyzate a C₈-C₁₈-alkyl-di-C₁-C₃-alkyl hydroxypropyl wheat protein hydrolyzate.

17. Tinting shampoo according to claim 16, comprising as cationic plant protein hydrolyzate lauryl dimethyl ammonium hydroxypropyl wheat protein hydrolyzate.

18. Tinting shampoo according to one or more of claims 10 to 17, comprising as nonionic surfactant 1% to 10% by weight, calculated to the total composition, of at least one C₈-C₁₈-alkyl polyglucoside with a condensation degree from 1.2 to 2.5.

19. Use of a cationic plant protein hydrolyzate in tinting shampoos comprising direct-acting hair dyestuffs and surfactants.
